(19) Europäisches Patentamt
European Patent Office
Office européen des brevets

(11) Publication number: **0 088 637**
**B1**

(12) **EUROPEAN PATENT SPECIFICATION**

(45) Date of publication of patent specification: **06.08.86**

(51) Int. Cl.$^4$: **C 12 P 33/06,** C 07 J 9/00

(21) Application number: **83301303.0**

(22) Date of filing: **09.03.83**

(54) **A method for producing ursodeoxycholic acid.**

(30) Priority: **09.03.82 JP 37116/82**

(43) Date of publication of application:
**14.09.83 Bulletin 83/37**

(45) Publication of the grant of the patent:
**06.08.86 Bulletin 86/32**

(84) Designated Contracting States:
**AT BE CH DE FR GB IT LI LU NL SE**

(58) References cited:
**US-A-4 303 754**

**CHEMICAL ABSTRACTS, vol. 89, no. 13, 23rd September 1978, page 623, no. 105865b, Columbus, Ohio, USA G. DEFAYE et al.: "Microbiological 7- and 15-hydroxylations of C-19 steroids"**

**CHEMICAL ABSTRACTS, vol. 98, no. 7, 14th February 1983, page 514, no. 51856q, Columbus, Ohio, USA H. SAWADA et al.: "Microbial production of ursodeoxycholic acid from lithocholic acid by fusarium equiseti M41"**

(73) Proprietor: **KABUSHIKI KAISHA YAKULT HONSHA**
**1-19, Higashishinbashi 1-chome**
**Minato-ku Tokyo 105 (JP)**

(72) Inventor: **Sawada, Haruji**
**16-8, 3-chome Fujimidai Kunitachi**
**Tokyo (JP)**
Inventor: **Taguchi, Hisaharu**
**11-18, Higashitoyonaka-cho Toyonaka**
**Osaka (JP)**

(74) Representative: **Brewer, Leonard Stuart et al**
**Sanderson & Co. 97 High Street**
**Colchester Essex CO1 1TH (GB)**

**EP 0 088 637 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a method for producing ursodeoxycholic acid, and more specifically to a method for producing ursodeoxycholic acid, which comprises subjecting lithocholic acid, the starting material, to the action of an ursodeoxycholic acid producing microorganism.

BACKGROUND OF THE INVENTION

Ursodeoxycholic acid, the final product of a method in accordance with the present invention, is a bile acid wherein hydroxyl groups are located at the 3α and 7β positions of the steroid nucleus contained therein, and has the structural formula (I) shown below.

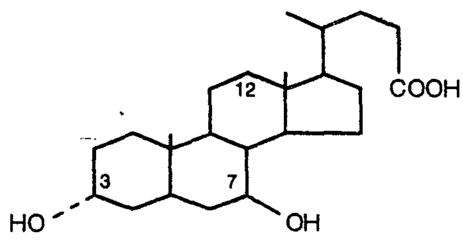

(I)

Ursodeoxycholic acid

Ursodeoxycholic acid is known as one of the traditional cholagogues. Since it was discovered that the compound is effective to solubilize cholesterol gallstones, much clinical data have been reported to show that the material is effective in the therapy of cholesterol gallstones by *per os* administration.

Since ursodeoxycholic acid occurs in the bile of a bear, it is possible to extract it therefrom. It is unrealistic, howevër, to assume that the supply is convenient or sufficient to meet the entire medical demands particularly from quantitative viewpoints. Accordingly, chemical synthesis processes have been employed for producing ursodeoxycholic acid.

One prior art example of the synthesis of ursodeoxycholic acid will be described below.

The first step is to methylate the carboxyl group of cholic acid (II), the starting material, thus converting cholic acid (II) to methyl cholate (III), as shown below.

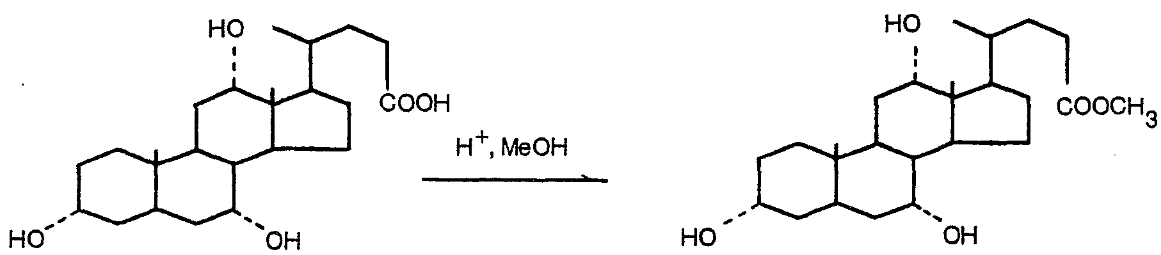


Cholic acid

(II)

Methyl cholate

(III)

The second step is to acetylate the hydroxyl groups located at the 3α and 7α positions of methyl cholate (III) by adding acetic anhydride to the same to obtain 3α, 7α-diacetoxy-12α-hydroxy-5β-methyl cholanate (IV), as shown below.

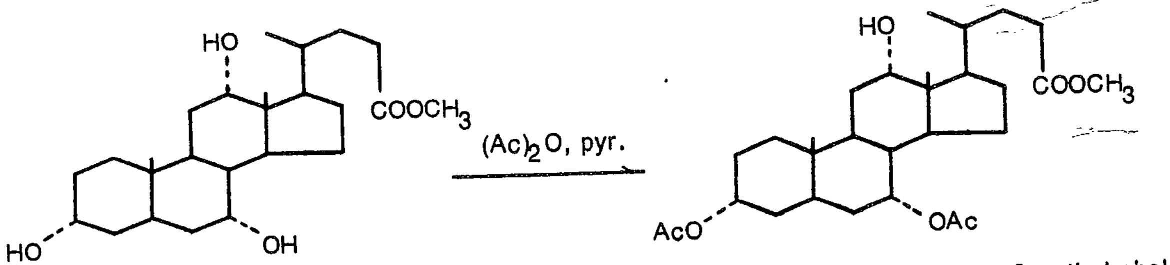


Methyl cholate

(III)

3α, 7α-Diacetoxy-12α-hydroxy-5β-methyl cholanate

(IV)

The third step is to oxidize the hydroxyl group located at the 12 position of 3α, 7α-diacetoxy-12α-hydroxy-5β-methyl cholanate (IV) by adding chromic acid to the same to obtain 3α, 7α-diacetoxy-12-keto-5β-methyl cholanate (V), as shown below.

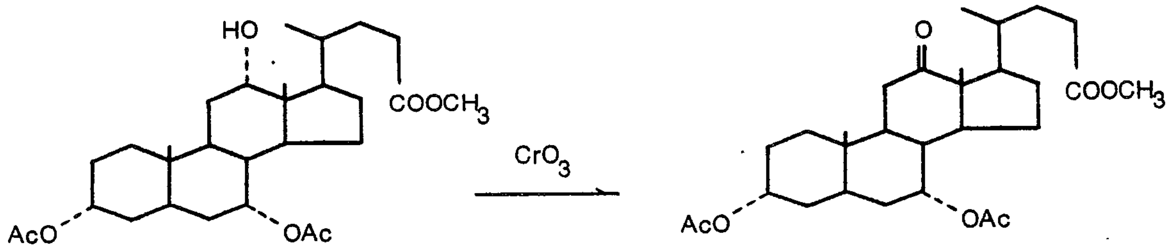


3α, 7α-Diacetoxy-12α-hydroxy-5β-methyl cholanate

(IV)

3α, 7α-Diacetoxy-12-keto-5β-methyl cholanate

(V)

The fourth step is to saponify 3α, 7α-diacetoxy-12-keto-5β-methyl cholanate (V) to obtain 12-ketochenodeoxycholic acid (VI), as shown below.

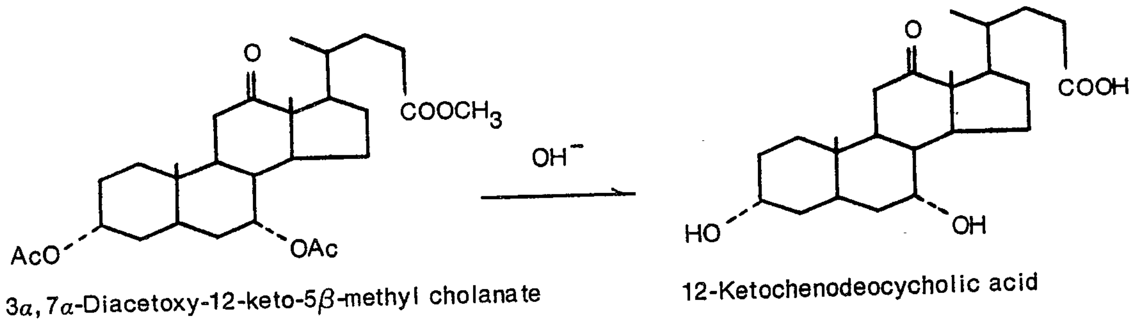


3α, 7α-Diacetoxy-12-keto-5β-methyl cholanate

(V)

12-Ketochenodeocycholic acid

(VI)

The fifth step is to reduce the carbonyl group located at the 12 position of 12-ketochenodeoxycholic acid (VI) by employing the Huang-Minlon modification to obtain chenodeoxycholic acid (VII), as shown below.

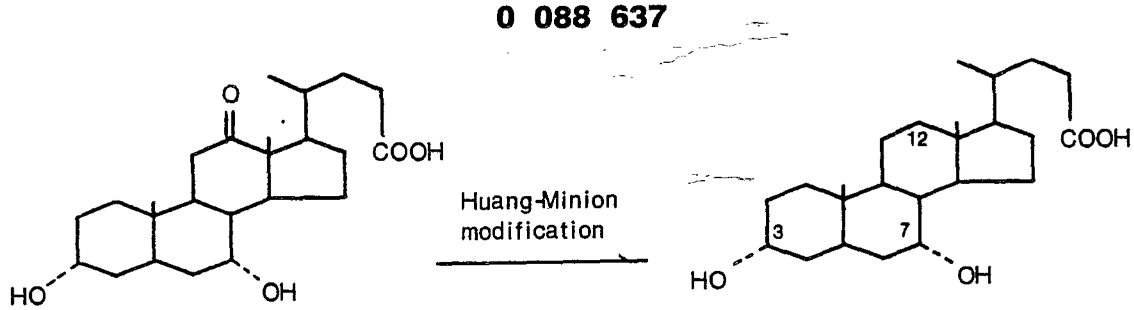


12-Ketochenodeoxycholic acid (VI) → Chenodeoxycholic acid (VII)

The sixth step is to oxidize the hydroxyl group located at the 7α position of chenodeoxycholic acid (VII) by adding N-bromosuccinimide to the same, thus obtaining 7-ketolithocholic acid (VIII), as shown below.

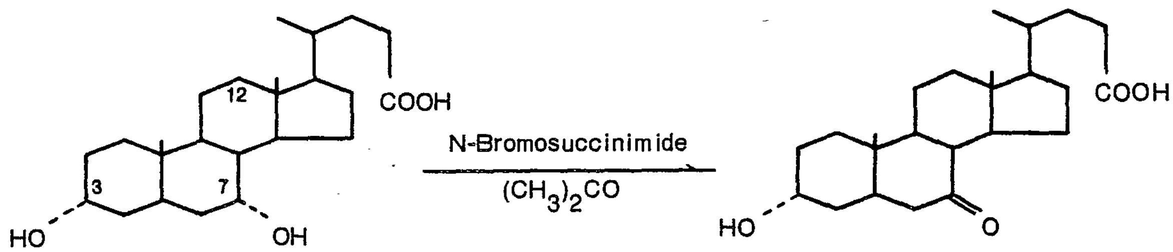


Chenodeoxycholic acid (VII) → 7-Ketolithocholic acid (VIII)

The seventh and final step is to reduce 7-ketolithocholic acid (VIII) by catalytic reduction carried out in n-butanol, thus obtaining the final product, ursodeoxycholic acid (I), as shown below.

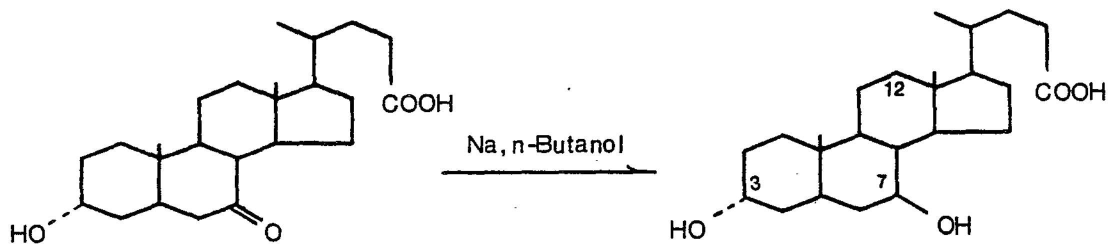


7-Ketolithocholic acid (VIII) → Ursodeoxycholic acid (I)

Since the aforementioned prior art process for producing ursodeoxycholic acid comprises 7 independent steps each of which requires a purification treatment thereafter, the process has various drawbacks such as its complexity and lower productivity.

Further, the process results in a decreased yield caused by various undesirable side reactions which are likely to accompany each of the independent steps.

Saltzman has disclosed in U.S. Patent No. 3,954,562 a one-step method for producing 12-dihydro steroids, typically chenodeoxycholic acid, by means of a microbial transformation technique which comprises subjecting 12-hydroxy steroids, typically cholic acid, to the action of a 12-dehydroxylase producing microorganism e.g. a microorganism belonging to *Clostridium* or *Bifidobacterium*. Sawada has disclosed in the Japanese Patent Application which was laid open to public inspection with the Toku-Kai-Sho No. 56—51997 a one-step method for producing 12-ketochenodeoxycholic acid which comprises subjecting dehydrocholic acid to the action of a microorganism belonging to *Lactobacileus*. However, neither of these methods available in the prior art refers to the production of ursodeoxycholic acid.

As far as we know, no method is available in the prior art for producing ursodeoxycholic acid by means of a microbial transformation technique.

OBJECTS AND SUMMARY OF THE INVENTION

An object of the present invention is to provide a one-step method for producing ursodeoxycholic acid from lithocholic acid, the starting material, by means of a microbial transformation technique, which overcomes the aforementioned drawbacks such as complexity, lower productivity and lower yield.

The invention provides a method for producing ursodeoxycholic acid (I), which method comprises subjecting lithocholic acid (IX) to the action of the microorganism *Fusarium equiseti* strain M—41 (deposition number FERM—P6351=FERM BP—259), whereby the hydrogen atom located at the 7β position of lithocholic acid (IX) is replaced by a hydroxyl group, thus producing ursodeoxycholic acid (I).

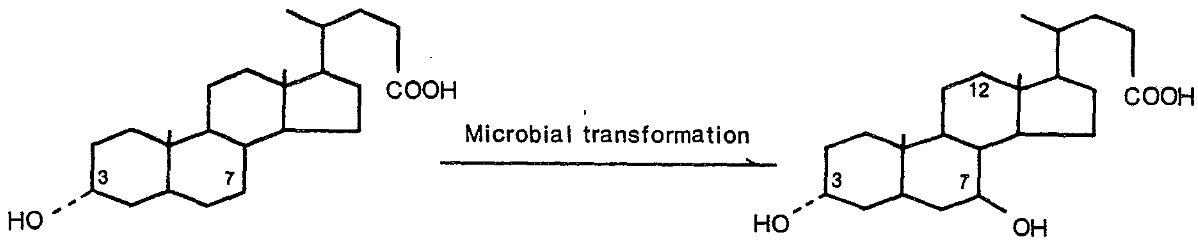


Lithocholic acid (IX) Ursodeoxycholic acid (I)

More specifically, the invention provides a method for producing ursodeoxycholic acid (I) by means of a microbial transformation technique comprising (A) a step of cultivating the microorganism *Fusarium equiseti* strain M—41 (FERM—P6351=FERM BP—259) (hereinafter referred to as Strain M—41), (B) a step of bringing lithocholic acid, the starting material, into contact with the microorganism (including any part thereof, such as spores, conidia, mycelia etc.) in any manner (in any type of medium, usually liquid, such as a nutrient medium, a reaction medium *et al.*), and (C) a step of recovering ursodeoxycholic acid, the final product, produced. The ursodeoxycholic acid as prepared according to the present invention is suitable for use in pharmaceutical compositions comprising a pharmaceutically acceptable carrier and for treating the human or animal body.

The invention also provides *Fusarium equiseti* strain M—41 (FERM—P6351=FERM BP—259). It was extracted from nature in ways which are conventional in themselves. We have discovered this particular microorganism and its valuable property of converting lithocholic acid to ursodeoxycholic acid.

The method of the invention can be carried out by subjecting the lithocholic acid to the action of the living ursodeoxycholic acid producing microorganism in any suitable way. For example, the lithocholic acid can be contacted with the microorganism which is then cultivated.

The method permits a wide variety of ways of bringing the lithocholic acid into contact with the microorganism. It is to be understood that where this specification refers to the microorganism, its effective parts such as spores can be employed unless the context requires otherwise.

The microbial transformation of the invention is usually carried out in a liquid, which can be any type of reaction liquid, including a nutrient medium, a reaction medium *et al.* In this specification, the nutrient medium is defined as a medium in which the microorganism is cultivated, and the reaction medium is defined as a medium which allows the microbial transformation to occur therein.

Preferred embodiments of the present method are:

(1) A method for producing ursodeoxycholic acid wherein step (B) of bringing lithocholic acid into contact with the microorganism (or any part thereof) occurs in a medium in which step (A) of cultivating the ursodeoxycholic acid producing microorganism is carried out.

(2) A method for producing ursodeoxycholic acid (a) which further comprises a step of harvesting the ursodeoxycholic acid producing microorganism and a step of preparing a reaction medium which contains the harvested microorganism (or any part thereof), and (b) in which step (B) of bringing lithocholic acid into contact with the microorganism (or any part thereof) occurs in this reaction medium.

(3) A method for producing ursodeoxycholic acid (a) in which step (A) of cultivating the ursodeoxycholic acid producing microorganism is carried out until spores are formed, (b) which further comprises a step of collecting the spores and a step of preparing a reaction medium which contains the collected spores, and (c) in which step (B) of bringing lithocholic acid into contact with the microorganism (or any part thereof) occurs in the reaction medium which contains the collected spores.

Embodiment (1) is directed to a method wherein the microbial action for producing ursodeoxycholic acid proceeds in a mixture of the ursodeoxycholic acid producing microorganism or its part e.g. spores, conidia and mycelia, the nutrient medium and lithocholic acid. Therefore, no limitation is imposed on the time at which lithocholic acid is added to the medium containing the ursodeoxycholic acid producing microorganism or part thereof.

Embodiment (2) is directed to a method wherein the microbial action for producing ursodeoxycholic acid proceeds in a mixture of the ursodeoxycholic acid producing microorganism or its part e.g. spores, conidia and mycelia, the reaction medium and lithocholic acid. The mixture can be either a solution or suspension. The microorganism can be employed either immediately after cultivation or after custody for some length of time in a fixed form which is readily realized by employment of a fixing agent such as calcium alginate etc.

It is noted that although embodiment (2) is originally a kind of batch process, a continuous process may be employed in which lithocholic acid is continuously introduced to be allowed contact with a lump of the fixed microorganism.

Embodiment (3) is directed to a method wherein the spores alone of the ursodeoxycholic acid producing microorganism are employed for the microbial action for producing the ursodeoxycholic acid. Some organic materials, typically some carbohydrates such as glucose, and casein hydrolysate etc., are effective to enhance the reaction, when they too are supplied to the reaction medium. The spores can be employed of course in a fixed form analogous to that of embodiment (2), and a continuous process can be employed analogous to that of embodiment (2).

BRIEF DESCRIPTION OF THE DRAWING

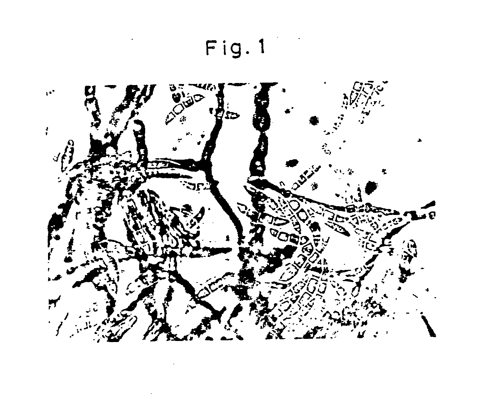

The present invention, together with its various features and advantages, can be readily understood from the following more detailed description presented in conjunction with the drawing, Fig. 1.

Fig. 1 is a reproduction of a photograph showing the appearance of Strain M—41 (FERM—P6351=FERM—BP-259), a microorganism belonging to *Fusarium equiseti*, the strain which is the ursodeoxycholic acid producing microorganism in accordance with the present invention.

DETAILED DESCRIPTION

(A) Identification of Strain M—41 (FERM—P6351=FERM BP-259)

In the early phase of cultivation of Strain M—41 carried out at 30°C on a potato dextrose agar medium, a single phialide grows on the side of a conidiophore, before macroconidia grow on the phialide.

The size (specifically the dimensions of the cross-sectional area of the phialide) ranges from 2.5 × 10 µm to 3 × 12.5 µm.

Sporodochia never grow on the conidiophore. In two weeks, the end of the conidiophore splits in the form of a broom, and 2 to 4 phialides grow at the split end of the conidiophore. The phialides having an obclavate shape are slightly larger than the phialides which previously grew at the side of a conidiophore, and the size of the phialides ranges from 3 × 12 µm to 4 × 17 µm.

The macroconidia have a variety of shapes including a spindle and a crescent. The macroconidia are slim and both their ends become narrower and terminate with sharp ends. Most marcoconidia have 5 septa and have the size range 3.8 × 50 µm to 4.5 × 63 µm. A few macroconidia have 3 septa and have the size range 3.8 × 30 µm to 4.8 × 38 µm.

It is quite seldom that microconidia grow.

Spherical or oval chlamydospores are formed on a mycelium. The spherical ones have the diameter range 7—9 µm, and the oval ones have their longer diameter ranging from 11 to 12.5 µm and their shorter diameter ranging from 5 to 8.7 µm.

Four-day cultivation carried out on a potato dextrose agar medium allows Strain M—41 to grow up to a colony whose diameter is 4.4 cm at 31°C, 5.8 cm at 27°C and 5.5 cm at 25°C.

Two-month cultivation carried out on a corn agar medium, an oatmeal agar medium or a potato agar medium does not allow Strain M—41 to form ascocarps, ascus or ascospores.

Fig. 1 is a reproduction of a photograph wherein the magnification is 600 showing the appearance of Strain M—41 which was cultivated for 14 days on an oatmeal agar medium containing 30 grams of oatmeal, 20 grams of agar and 1 (one) litre of water and having a pH of 6.5.

In accordance with Booth's classification (Booth C, the Genus Fusarium: Commonwealth Mycological Institute, Kew, Surrey (1971)), Strain M—41 described above was identified as *Fusarium equiseti* and named *Fusarium equiseti* M—41. This is because the appearance of Strain M—41 is identical to the reference appearance of *Fusarium equiseti* (Corda) Sacc., Sylloge Fung., except that the length (5 µm) of some of the macroconidia having 5 septa is rather longer than that of Booth's reference. Strain M—41 has been deposited at the Fermentation Research Institute, Agency of Industrial Science & Technology, Japan with the Deposition No. FERM—P6351=FERM BP-259.

The physiological properties of Strain M—41 are as follows:

(1) Optimum growth conditions (pH and temperature). The optimum pH range is 6 to 6.5, and the optimum temperature range is 27 to 28°C.

(2) Allowable growth conditions (pH and temperature). The allowable pH range is 3.5 to 8, and the allowable temperature range is 10 to 37°C.

(3) Other specific feature.

In the last phase of cultivation carried out on a Czapek agar medium or in a liquid medium, the colour of spores of the strain becomes pale pink.

(B) Screening of Strain M—41 (FERM—P6351=FERM BP-259)

609 independent mould strains were isolated from the soil picked up at 6 independent places in and adjacent to Osaka Prefecture, Japan. Each of these strains was inoculated in 20 ml of a basal medium containing lithocholic acid which is kept in a 100-ml triangular flask to be incubated on a shaker at 28°C for 5 days. After completion of the incubation, the pH was adjusted to 3 with 5N hydrochloric acid, before bile acids were extracted with 50 ml of ethyl acetate. After being dehydrated with Glauber's salt, the extract was vacuumcondensed by means of a rotary evaporator. Qualitative analysis was applied to each of the condensed extracts by means of thin layer chromatography, in order to screen any strain which is capable of producing ursodeoxycholic acid. In the aforementioned thin layer chromatography, the bands for each of the condensed extracts were compared with that for standard ursodeoxycholic acid. In this manner, we were successful in screening a strain which is capable of producing ursodeoxycholic acid from lithocholic acid. The screened strain was named Strain M—41. The mould strain from which we were successful in screening Strain M—41 was isolated from the soil picked up at a hill in Minoo, Osaka Prefecture, Japan on July 28, 1981.

The basal medium employed for isolation of the strain contained 1 litre of water, 30 g of glucose, 3 g of sodium nitrate, 5 g of yeast extract, 2 g of monopotassium phosphate, 3 g of dipotassium phosphate, 0.5 g of magnesium sulphate, 0.5 g of calcium chloride, 20 mg of iron (II) sulphate and 0.5 g of lithocholic acid which is the substrate to be transformed.

(C) Example 1

A medium is prepared by mixing 1 litre of water with 50 g of glucose, 3 g of $NaNO_3$, 3 g of $K_2HPO_4$, 2 g of $KH_2PO_4$, 0.5 g of $MgSO_4 \cdot 7H_2O$, 0.5 g of KCl, 20 mg of $FeSO_4$ and 0.5 g of lithocholic acid. 6 litres of the medium is poured into a reaction tank having a capacity of 10 litres. Separately, Strain M—41 (FERM—P6351=FERM BP-259) is incubated on a shaker at 28°C for 24 hours employing the same medium as is described above to prepare a preculture. 0.3 litres of this preculture is inoculated in the aforementioned medium to be cultivated under aerobic conditions at 28°C for 3 days. During the cultivation, the mixture is stirred at the rate of 30 rpm, the pH is adjusted to 7, and air is supplied at the rate of 0.5 vvm. After completion of the cultivation, hydrochloric acid is added to the culture to adjust the pH to 3. Thereafter, an extraction process is applied to the culture three times with 2 volumes of ethyl acetate in order to extract (a) the ursodeoxycholic acid produced, and (b) the remaining lithocholic acid, out of the residual medium containing the ursodeoxycholic acid producing microorganism. The resultant ethyl acetate layer, which contains materials including (a) ursodeoxycholic acid and (b) lithocholic acid, is washed with water. Then, anhydrous Glauber's salt is added for the purpose of dehydration. Thereafter, ethyl acetate is evaporated off under vacuum to leave a residue containing (a) the ursodeoxycholic acid and (b) the remaining lithocholic acid. This residue is applied to a column containing 80 g of silica gel (Wako gel C—200), before there is applied to the column a mixture of solvents composed of chloroform, acetone and acetic acid at the volumetric ratio of 100:100:1 to elute ursodeoxycholic acid. Thereafter, the fractions which contain ursodeoxycholic acid in the aforementioned solvent mixture are selectively gathered. In this manner, the ursodeoxycholic acid is readily separated from the starting material, lithocholic acid. Further, a purification process is carried out in the following manner. The solvents are removed from the fraction by means of evaporation, leaving the ursodeoxycholic acid. The residue, containing predominantly the ursodeoxycholic acid is dissolved in a small quantity of ethanol. The ethanol solution is applied to a preparative thin layer of silica gel (Merck Kieselgel G—60, $F_{254}$, 2-mm thick) in order to develop the ursodeoxycholic acid employing the aforementioned mixture of solvents. After the developing process, the silica gel layer is dried, a band of the thin layer of silica gel containing ursodeoxycholic acid is sliced precisely, and the ursodeoxycholic acid is extracted therefrom with ethanol. The extract containing ursodeoxycholic acid is condensed, and the ursodeoxycholic acid is crystallized from a mixture of ethanol and water. A further purification process could be employed wherein ursodeoxycholic acid is recrystallized from an ethyl acetate solution.

The yield of ursodeoxycholic acid is 0.25 g from 1 litre of culture or 0.5 g of lithocholic acid, the starting material, representing 50 weight % yield of ursodeoxycholic acid from lithocholic acid.

(D) Example 2

An aerobic cultivation process similar to that which is described in Example 1 is employed to incubate Strain M—41 (FERM—P6351=FERM BP-259) in a reaction tank containing 6 litres of a medium (pH 7.0) prepared by mixing 1 litre of water with 50 g of oatmeal and 0.1 g of lithocholic acid. After the cultivation has continued for 24 hours, a further 0.5 g/litre of lithocholic acid is supplied, before a further cultivation is carried out for 48 hours.

After completion of the cultivation, a purification process identical to that which is described in Example 1 is employed to extract and isolate the produced ursodeoxycholic acid. The yield of ursodeoxycholic acid is 0.28 g from 1 litre of culture or 0.6 g of lithocholic acid, representing 47 weight % yield of ursodeoxycholic acid from lithocholic acid.

(E) Example 3

A medium is prepared by mixing 1 litre of water with 20 g of starch, 50 g of corn steep liquor, 5 g of beef

extract, 5 g of polypeptone, 5 g of sodium chloride and 0.5 g of lithocholic acid. Strain M—41 (FERM—P6351=FERM BP-259) is incubated in 6 litres of the medium contained in a reaction tank. Cultivation is carried out at 28°C for 36 hours, while the pH is maintained at 7.

After completion of the cultivation, the cultivated microorganism is collected by means of centrifugation. The collected microorganism is washed with a phosphate buffer (pH 7.0). Separately, a reaction medium (pH 7.0) is prepared by mixing 1 litre of water with 2 g of monopotassium phosphate, 3 g of dipotassium phosphate, 10 g of glucose and 0.2 g of lithocholic acid. The aforementioned collected microorganism is added to this reaction medium to make a suspension containing 30 g of the microorganism in 1 litre of the medium. A conversion reaction is carried out under aeration in this suspension at 28°C for 48 hours.

After completion of the conversion reaction, a purification process identical to that which is described in Example 1 is employed to extract and isolate the ursodeoxycholic acid produced. The yield of ursodeoxycholic acid is 0.1 g from 1 litre of reaction medium or 0.2 g of lithocholic acid, representing 50 weight % yield of ursodeoxycholic acid from lithocholic acid.

(F) Example 4

An agar medium (pH 7) is prepared by mixing 1 litre of water with 30 g of oatmeal, 0.2 g of lithocholic acid and 20 g of agar. Strain M—41 (FERM—P6351=FERM BP-259) is aerobically cultivated on the agar medium at 28°C for 2 weeks.

After completion of the cultivation, the cultivated microorganisms are suspended in a phosphate buffer (pH 7.0). After mycelia are filtered with a gauze filter out of the suspension, the spores remaining in the suspension are collected by means of centrifugation. The collected spores are washed with water.

The spores are poured in a phosphate buffer (pH 7.0) to produce a reaction medium containing $10^{10}$ spores in 1 millilitre thereof.

2 g of glucose and 0.2 g of lithocholic acid are added to 1 litre of the reaction medium to allow a conversion reaction to occur therein at 30°C for 48 hours under the asceptic aeration.

After completion of the conversion reaction, a purification process identical to that which is described in Example 1 is employed to extract and isolate the ursodeoxycholic acid produced. The yield of ursodeoxycholic acid is 0.1 g from 1 litre of the reaction medium or 0.2 g of lithocholic acid, representing 50 weight % yield of ursodeoxycholic acid from lithocholic acid.

(G) Identification of produced ursodeoxycholic acid with reference ursodeoxycholic acid

Firstly, thin layer chromatography (Merck Kieselgel G—60, $F_{254}$, 0.25-mm thick) was employed to identify each of the products of Examples 1 to 4 with the standard (ursodeoxycholic acid supplied by Gasukuro Kogyo Co., Ltd.). The Rf values determined for each of the products of Examples 1 to 4 were identical with the Rf values determined for the standard ursodeoxycholic acid for each of the developing solvent systems tabulated below.

| | Developing Solvent System | Rf Value |
|---|---|---|
| 1. | Diethyl ether:acetic acid (249:1) | 0.16 |
| 2. | Chloroform:acetone:acetic acid (100:100:1) | 0.49 |
| 3. | 2,2,4-trimethyl pentane:ethyl acetate: acetic acid: butanol (20:10:3:3) | 0.45 |
| 4. | 2,2,4-trimethyl pentane:isopropanol:acetic acid (60:20:0.5) | 0.36 |
| 5. | 2,2,4-trimethyl pentane:ethyl acetate:acetic acid (5:1:1) | 0.35 |
| 6. | Chloroform:methanol:acetic acid (80:12:3) | 0.75 |
| 7. | Chloroform:methanol:water (70:25:3) | 0.72 |
| 8. | 2,2,4-trimethyl pentane:diisopropyl ether:acetic acid: butanol:water (10:5:5:3:1) | 0.36 |
| 9. | Chloroform:ethyl acetate:acetic acid (9:9:2) | 0.54 |
| 10. | 2,2,4-trimethyl pentane:acetic acid:diisopropyl ether:isopropanol (10:6:5:1) | 0.51 |
| 11. | Chloroform:methanol:7N $NH_4OH$:water (21:15:1:2) | 0.78 |
| 12. | 2,2,4-trimethyl pentane:diisopropyl ether: acetic acid:isopropanol (2:1:1:1) | 0.77 |
| 13. | Isopropanol:ethyl acetate:water:$NH_4OH$ (20:25:6:4) | 0.38 |
| 14. | 2,2,4-trimethyl pentane:diisopropyl ether: acetic acid:butanol:isopropanol:water (10:5:5:3:6:1) | 0.80 |
| 15. | Propanol:acetic acid:water (95:4:1) | 0.87 |
| 16. | Butanol:acetic acid:water (100:7:5) | 0.89 |
| 17. | Ethyl acetate:methanol:acetic acid (7:2:1) | 0.87 |

Additionally, various analyses tabulated below were applied to each of the products of Examples 1 to 4 to identify each of them with the aforementioned standard ursodeoxycholic acid.

1. Elementary analysis
2. Melting point test and mixed melting point test
3. Infrared spectrum analysis
4. Mass spectrum analysis
5. Nuclear magnetic resonance spectrum analysis.

The results of all the foregoing tests determined that the property of each of the products of Examples 1 to 4 is identical with that of the standard ursodeoxycholic acid.

(H) Conclusion

The foregoing description shows that a one-step method for the production of ursodeoxycholic acid from lithocholic acid by means of microbial transformation is successfully provided and that the method in accordance with the present invention is free from the draw-backs inherent in any method available in the prior art, namely complexity of process, lower productivity and lower yield. In other words, the method in accordance with the present invention has various features including simplicity in process, high productivity and high yield.

Various modifications of the described embodiment will become apparent to persons skilled in the art upon reference to the description of the present invention.

**Claims**

1. A method for producing ursodeoxycholic acid, which method comprises subjecting lithocholic acid to the action of the microorganism *Fusarium equiseti* strain M—41 (deposition number FERM—P6351=FERM BP-259).

2. A method for producing ursodeoxycholic acid by microbial transformation comprising:

(A) a step of cultivating the microorganism *Fusarium equiseti* strain M—41 (deposition number FERM—P6351=FERM BP-259);

(B) a step of bringing lithocholic acid into contact with the microorganism; and

(C) a step of recovering the ursodeoxycholic acid produced.

3. A method according to claim 2 wherein step (B) of bringing lithocholic acid into contact with the microorganism occurs in a medium in which step (A) of cultivating the microorganism is carried out.

4. A method according to claim 2 (a) which further comprises a step of harvesting the microorganism and a step of preparing a reaction medium which contains the harvested microorganism, and (b) in which step (B) of bringing lithocholic acid into contact with the microorganism occurs in this reaction medium.

5. A method according to claim 2 (a) in which step (A) of cultivating the microorganism is carried out until spores are formed, (b) which further comprises a step of collecting the spores and a step of preparing a reaction medium which contains the collected spores, and (c) in which step (B) of bringing lithocholic acid into contact with the microorganism occurs in the reaction medium which contains the collected spores.

6. A method for producing ursodeoxycholic acid by microbial transformation comprising:

(A) a step of preparing a reaction liquid which contains a part of the microorganism *Fusarium equiseti* strain M—41 (deposition number FERM—P6351=FERM BP-259);

(B) a step of bringing lithocholic acid into contact with the reaction liquid; and

(C) a step of recovering the ursodeoxycholic acid produced.

7. A method according to claim 6 wherein the part of the microorganism is its spores.

8. *Fusarium equiseti* strain M—41 (deposition number FERM—P6351=FERM BP-259).

**Patentansprüche**

1. Verfahren zur Herstellung von Ursodeoxycholsäure wobei man Lithocholsäure der Einwirkung des Mikroorganismus *Fusarium equiseti* Art M—41 (Depotnummer FERM—P6351=FERM BP-259) aussetzt.

2. Verfahren zur Herstellung von Ursodeoxycholsäure durch mikrobische Umsetzund bestehend aus:

(A) einer Stufe für die Züchtung des Mikroorganismus *Fusarium equiseti* Art M—41 (Depotnummer FERM—P6351=FERM BP-259),

(B) einer Stufe wobei die Lithocholsäure in Berührung mit dem Mikroorganismus gebracht wird, und

(C) einer Stufe wobei die hergestellte Ursodeoxycholsäure gewonnen wird.

3. Verfahren nach Anspruch 2, wobei als Stufe (B) die Lithocholsäure in Berührung mit dem Mikroorganismus gebracht wird, in einem Mittel in welchem die Stufe (A) der Züchtung des Mikroorganismus durchgeführt wird.

4. Verfahren nach Anspruch 2 (a) welches eine Stufe für das Ernten des Mikroorganismus umfasst und eine Stufe worin ein den geernteten Mikroorganismus enthaltendes Reaktionsmittel vorbereitet wird, und (b) worin als Stufe (B) Lithocholsäure in Berührung mit dem Mikroorganismus in diesem Reaktionsmittel gebracht wird.

5. Verfahren nach Anspruch 2 (a) worin die Stufe (A) der Züchtung des Mikroorganismus bis zur Bildung von Sporen durchgeführt wird, und (b) welches besteht aus einer Stufe für die Sammlung der Sporen und einer Stufe für die Vorbereitung eines die gesammelten Sporen enthaltendes Reaktionsmittels, und (c) worin als Stufe (B) die Lithocholsäure in dem Reaktionsmittel, welches die gesammelten Sporen enthält, mit dem Mikroorganismus in Berührung gebracht wird.

6. Verfahren zur Herstellung von Ursodeoxycholsäure durch mikrobische Umsetzung bestehend aus:

(A) einer Stufe worin eine Reaktionsflüssigkeit vorbereitet wird, welche einen Teil des Mikroorganismus *Fusarium equiseti* Art M—41 (Depotnummer FERM—P6351=FERM BP-259) enthält,

(B) einer Stufe wobei Lithocholsäure in Berührung mit der Reaktionsflüssigkeit gebracht wird, und

(C) einer Stufe worin die hergestellte Ursodeoxycholsäure gewonnen wird.

7. Verfahren nach Anspruch 6, worin der Teil des Mikroorganismus dessen Sporen sind.

8. *Fusarium equiseti* Art M—41 (Depotnummer FERM—P6351=FERM BP-2591).

**Revendications**

1. Méthode pour produire l'acide ursodéoxycholique comprenant la soumission d'acide lithocholique à l'action du micro-organisme *Fusarium equiseti* souche M—41 (numéro déposé FERM—P6351=FERM BP-259).

2. Méthode pour produire l'acide ursodéoxycholique par transformation microbienne constituée par: (A) un stade de cultivation du micro-organisme *Fusarium equiseti* souche M—41 (numéro déposé FERM—P6351=FERM BP-259); (B) un stade de mise en contact de l'acide lithocholique avec le micro-organisme: et (C) un stade de récuperation de l'acide ursodéoxycholique ainsi produit.

3. Méthode selon la revendication 2 ou le stade (B) de mise en contact de l'acide lithocholique avec le micro-organisme se fait dans un milieu ou le stade (A) de cultivation du micro-organisme a lieu.

4. Méthode selon la revendication 2 (a) comprenant en outre un stade de récolte du micro-organisme et un stade de préparation d'un milieu de réaction contenant le microorganisme récolté et (b), dans laquelle le stade (B) de mise en contact de l'acide lithocholique avec le micro-organisme se fait dans ce milieu de reaction.

5. Méthode selon la revendication 2 (a) dans laquelle la stade (a) de cultivation du micro-organisme s'étend jusqu'à la formation de spores, (b) qui comprend en outre un stade de recueillement des spores et un stade de préparation d'un milieu de réaction contenant les spores recueillies, et (c) dans laquelle le stade (B) de mise en contact de l'acide lithocholique avec le micro-organisme se fait dans le milieu de réaction contenant les spores recueillies.

6. Méthode pour produire l'acide ursodéoxycholique par transformation microbienne comprenant:

(A) un stade de préparation d'un liquide de réaction contenant une partie du micro-organisme *Fusarium equiseti* souche M—41 (numéro déposé FERM—P6351=FERM BP-250)

(B) un stade de mise en contact de l'acide lithocholique avec le liquide de réaction: et

(C) un stade de récuperation de l'acide ursodéoxycholique produit.

7. Méthode selon la revendication 6, dans laquelle la partie du micro-organisme est ses spores.

8. *Fusarium equiseti* souche M—41 (numéro déposé FERM—P6351=FERM BP-259).

Fig. 1